# FASCICULE DE BREVET EUROPEEN

(11) **EP 3 509 704 B1**
(45) Date de publication et mention de la délivrance du brevet: **30.03.2022**
(21) Numéro de dépôt: 17762149.7
(22) Date de dépôt: 12.09.2017
(51) Int. Cl.: A61Q 5/12, A61K 8/73

(54) **PROCEDE DE TRAITEMENT DES MATIERES KERATINIQUES A PARTIR DE POLYSACCHARIDE AMINE ET OXYDE**
VERFAHREN ZUR BEHANDLUNG VON KERATINÖSEN MATERIALIEN UNTER VERWENDUNG VON AMINIERTEM UND OXIDIERTEM POLYSACCHARID
METHOD FOR TREATING KERATINOUS MATERIALS USING AMINATED AND OXIDISED POLYSACCHARIDE

(30) Priorité: 12.09.2016 FR 1658478
(43) Date de publication de la demande: 17.07.2019
(73) Titulaire: L'Oreal, 75008 Paris (FR)
(72) Inventeur: PHILIPPE, Michel, 93601 Aulnay-Sous-Bois (FR); JEGOU, Gwenaëlle, 93601 Aulnay-Sous-Bois (FR)
(74) Mandataire: L'Oreal
(86) Numéro de dépôt international: PCT/EP2017/072828
(87) Numéro de publication internationale: WO 2018/046747

(56) Documents cités:
- WO-A1-2013/132062
- FR-A1- 2 842 200
- US-A1- 2002 082 399
- US-A1- 2003 053 977
- US-A1- 2011 224 724
- AYMAN KARAM ET AL: "Rational Design of Sugar-Based-Surfactant Combined Catalysts for Promoting Glycerol as a Solvent", CHEMISTRY - A EUROPEAN JOURNAL, vol. 14, no. 33, 17 novembre 2008 (2008-11-17), pages 10196-10200, XP055355900, ISSN: 0947-6539, DOI: 10.1002/chem.200801495
- FREDON E ET AL: "Hydrophobic films from maize bran hemicelluloses", CARBOHYDRATE POLYMERS, APPLIED SCIENCE PUBLISHERS, LTD. BARKING, GB, vol. 49, no. 1, 1 juillet 2002 (2002-07-01), pages 1-12, XP004341983, ISSN: 0144-8617, DOI: 10.1016/S0144-8617(01)00312-5

## Description

L'invention concerne i) un procédé de traitement des matières kératiniques, en particulier les fibres kératiniques, plus particulièrement des fibres kératiniques humaines telles que les cheveux, mettant en oeuvre a) au moins un polysaccharide aminé et oxydé comprenant au moins un groupe carboxy(late) en particulier au moins une inuline aminée et oxydée à groupe(s) carboxy ou carboxylate(s) ; ii) une composition comprenant au moins l'ingrédient a) ; iii) l'utilisation d'au moins un ingrédient a) pour traiter les fibres kératiniques, en particulier les fibres kératiniques humaines telles que les cheveux ; et iv) un kit ou dispositif à plusieurs compartiments comprenant au moins un ingrédient a).

Les cheveux sont généralement abîmés et fragilisés par l'action des agents atmosphériques extérieurs tels que la lumière, le soleil et les intempéries, ainsi que par les traitements mécaniques ou chimiques tels que le brossage, le peignage, les teintures, les décolorations, les permanentes, les défrisages, les lavages répétés. Les cheveux se retrouvent ainsi endommagés par ces différents facteurs et peuvent à la longue devenirs secs, rêches, cassants ou ternes ou fourchus ou mous.

Ainsi, pour remédier à ces inconvénients, il est usuel d'avoir recours à des soins capillaires qui mettent en oeuvre des compositions destinées à conditionner les cheveux de manière convenable en leur apportant des propriétés cosmétiques satisfaisantes, notamment du lissage, de la brillance, de la douceur au toucher (un toucher naturel, les cheveux ne sont plus rêches), de la souplesse, de la légèreté, de bonnes propriétés de démêlage induisant une facilité au peignage, une bonne discipline des cheveux qui ont ainsi une bonne mise en forme.

Ces compositions de soins capillaires peuvent être, par exemple, des shampooings conditionneurs, des après shampooings, des masques, des sérums. Toutefois, l'effet conditionneur obtenu s'estompe au cours des shampoings successifs et ne présente pas une rémanence au shampooing satisfaisante.

Il est connu de mettre en oeuvre des compositions de soin comprenant des sucres réducteurs, tels que des monosaccharides, utilisés en tant qu'agents de conditionnement, pour notamment réparer les fibres kératiniques qui ont été endommagées par des traitements agressifs.

En effet, la demande de brevet US 2002/0193264 décrit un procédé pour le conditionnement des fibres kératiniques, dans lequel on applique sur lesdites fibres au moins un sucre choisi parmi des monosaccharides et on met en oeuvre une étape de chauffage des fibres kératiniques. De la même façon, la demande de brevet US 2002/0172653 divulgue un procédé pour le conditionnement des fibres kératiniques comprenant une étape d'application sur lesdites fibres d'un sucre choisi parmi des monosaccharides particuliers en C₅-C₇ et une étape de chauffage des fibres kératiniques. Toutefois, la mise en oeuvre de sucres réducteurs suivie d'un traitement thermique peuvent entrainer une modification non souhaitée de la couleur des fibres kératiniques. De plus, les sucres réducteurs se dégradent facilement, notamment sous l'action de shampooings, ce qui a pour conséquence que les propriétés cosmétiques conférées aux fibres ne sont pas rémanentes. Ainsi les fibres kératiniques ne sont pas traitées de manière durable en particulier pas protégées, réparées ou transformées cosmétiquement.

Dans le domaine de la coloration, on connaît de la demande de brevet FR 2 944 967 l'utilisation de polysaccharides oxydés pour protéger la couleur des fibres kératiniques qui ont été teintes artificiellement. On connait également de la demande internationale WO 2013/132062 un procédé de traitement des cheveux, qui consiste à mettre en oeuvre un ou plusieurs polysaccharides oxydés et à élever la température des fibres kératiniques. La demande de brevet US 2003-0053977 décrit en outre un procédé pour protéger les fibres kératiniques qui met en oeuvre de la chaleur et une glucosamine.

Néanmoins les résultats obtenus ne sont toujours satisfaisants en termes de discipline, de réparation et/ou de protection des fibres kératiniques en particulier les cheveux abimés et/ou sensibilisés, en termes de douceurs de surfaces desdits fibres en particulier au niveau des pointes à sec, ou pour démêler les fibres kératiniques mouillées ou humides.

Par ailleurs il est recherché de nouveaux matériaux réactifs utilisables en cosmétique obtenus par des procédés respectueux de l'environnement. En particulier, il est d'intérêt de disposer de nouveaux polymères multi-réactifs capable de se fixer de manière durable aux substrats kératiniques de manière à apporter un bénéfice durable à ces substrats. En effet, il n'existe pas de matériaux verts aminés d'origine naturelle qui possèdent d'autres fonctions réactives oxydées.

Il existe donc un réel besoin de développer une composition et un procédé de traitement des fibres kératiniques telles que les cheveux qui soit capable de traiter de manière durable notamment de conditionner, de discipliner et/ou de protéger les cheveux et ceci sans entraîner une modification de leur couleur. Il est également intéressant de trouver un moyen pour traiter les fibres kératiniques abimées en les réparant, c'est-à-dire en améliorant la cosmétique des fibres kératiniques de façon intrinsèque, de diminuer la casse des fibres kératiniques et/ou de prévenir la casse des fibres kératiniques.

Ce(s) problème(s) technique(s) est(ont) été résolu(s) par le procédé de traitement des matières kératiniques, en particulier des fibres kératiniques, notamment humaines, de préférence des cheveux, comprenant une étape consistant à appliquer sur lesdites fibres a) un ou plusieurs polysaccharide(s) aminé(s) oxydé(s) comprenant au moins un groupe carboxy(late), de préférence le ou lesdits polysaccharide(s) est(sont) non ionique(s) ou anionique(s).

L'invention a également pour objet une composition comprenant un ou plusieurs polysaccharide(s) aminé(s) oxydé(s) comprenant au moins un groupe carboxy(late), de préférence le ou lesdits polysaccharide(s) est(sont) non ionique(s) ou anionique(s).

Un autre objet de l'invention est l'utilisation des ingrédients a) pour améliorer la discipline, le conditionnement des fibres kératiniques, pour réparer les fibres kératiniques abimées, et/ou pour prévenir la casse des fibres kératiniques.

En particulier, les cheveux traités avec le procédé selon l'invention restent disciplinés car on ne constate pas la présence de frisottis. Ainsi les cheveux sont alignés, lisses et se démêlent aisément ce qui les rend plus facile à peigner. Les cheveux traités ont également plus de corps (ils ne sont pas mous), et sont donc plus faciles à coiffer. Les cheveux traités ont une bonne mise en forme. Par ailleurs, les cheveux traités sont également plus brillants et plus doux au toucher. Ils sont moins cassants et plus résistants.

Après traitement, les cheveux ne sont pas chargés et présentent un toucher naturel.

Le procédé selon l'invention a l'avantage de conférer une bonne rémanence après un shampooing de ces bonnes propriétés cosmétiques de conditionnement du cheveu. Aussi, les cheveux traités sont conditionnés de manière durable.

Dans ce qui va suivre à moins qu'une mention contraire soit dite :
▪ par « *sel d'acide organique ou minéral* » on entend des sels d'acide organique ou minéral cosmétiquement acceptables, plus particulièrement les sels choisis parmi un sel dérivé i) d'acide chlorhydrique HCl, ii) d'acide bromhydrique HBr, iii) d'acide sulfurique H₂SO₄, iv) d'acides alkylsulfoniques : Alk-S(O)₂OH tels que d'acide méthylsulfonique et d'acide éthylsulfonique ; v) d'acides arylsulfoniques : ArS(O)₂OH tel que d'acide benzène sulfonique et d'acide toluène sulfonique ; vi) d'acide citrique ; vii) d'acide succinique ; viii) d'acide tartrique ; ix) d'acide lactique, x) d'acides alkoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique H₃PO₄; xiii) d'acide acétique CH₃C(O)OH ; xiv) d'acide triflique CF₃SO₃H et xv) d'acide tétrafluoroborique HBF₄;
▪ par « *contre ion cationique* » on entend des contre-ions cationiques cosmétiquement acceptables, particulièrement choisis parmi les cations de métaux alcalins ou alcalino-terreux tels que le sodium, potassium, ou calcium, ammonium R,R',R"N⁺-, ou phosphonium R,R',R"P⁺- ; avec R, R' et R", identiques ou différents, représentent un atome d'hydrogène, un groupe (C₁-C₆)alkyle, éventuellement substitué notamment par un groupe hydroxy tel que hydroxyéthyle ;
▪ par « *alkyle* », un radical linéaire ou ramifié contenant de 1 à 14 atomes de carbone, en particulier de 1 à 8 atomes de carbone, de préférence de 1 à 6 atomes de carbone, plus préférentiellement de 1 à 4 atomes de carbone, par exemple méthyle, éthyle, n-propyle, iso-propyle, butyle, n-pentyle, n-hexyle, de préférence méthyle ;
▪ par alcoxy on entend un groupe alkyle oxy avec « *alkyle* », tel que défini précédemment ;
▪ par « *éventuellement substitué* » suivi de (C₁-C₆)alkyle on entend que ledit groupe alkyle peut être substitué par un ou plusieurs, identiques ou différents, groupes choisis parmi i) hydroxy, ii) halogène, iii) (C₁-C₄)alkoxy, iv) hydroxy(C₂-C₄)alcoxy ; v) (di)(hydroxy(C₁-C₄)(alkyl)amino, vi) Rₐ-Zₐ-C(Z_{b})-Z_{c}-, et v) Rₐ-Zₐ-S(O)ₜ-Z_{c}- avec Zₐ, et Z_{b}, identiques ou différents, représentant un atome d'oxygène, de soufre, ou un groupe NRₐ', Z_{c}, représentant une liaison, un atome d'oxygène, de soufre, ou un groupe NRₐ ; Rₐ, représentant un atome d'hydrogène, un groupement (C₁-C₄)alkyle et R_{a'} représentant un atome d'hydrogène ou un groupement alkyle et t vaut 1 ou 2 ;
▪ un radical « *aryle* » représente un groupement carboné mono ou polycyclique, condensé ou non, comprenant de 6 à 22 atomes de carbone, et dont au moins un cycle est aromatique ; particulièrement le radical aryle est un phényle, biphényle, naphtyle, indényle, anthracényle, ou tétrahydronaphtyle, de préférence phényle ;
▪ un « *radical hétéroaryle* » représente un groupement mono ou polycyclique, condensé ou non, , comprenant de 5 à 22 chaînons, de 1 à 6 hétéroatomes choisis parmi l'atome d'azote, d'oxygène, et de soufre, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tetrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle ;
▪ par ailleurs, les sels d'addition utilisables dans le cadre de l'invention sont notamment choisis parmi les sels d'addition avec une base cosmétiquement acceptable tels que les agents alcalinisants tels que définis ci-après comme les hydroxydes de métal alcalin comme la soude, la potasse, l'ammoniaque, les amines ou les alkanolamines ;
▪ par « (hétéro)cyclo(C₅-C₇)alkyle » on entend un groupement mono ou bicyclique, , condensé ou non, de préférence monocyclique saturé ou renfermant une ou plusieurs insaturations éthyléniques, comprenant de 5 à 7 chaînons, comprenant dans au moins un cycle un hétéroatome N, pouvant renfermer de 1 à 3 hétéroatomes non adjacents additionnels choisis parmi l'atome d'azote, d'oxygène, et de soufre, cet hétérocycle pouvant être substitué par un ou plusieurs radicaux , identiques ou différents choisis parmi les radicaux alkyle, hydroxyalkyle, alcoxy préférentiellement , selon la présente invention, l'hétérocycle saturé ou insaturé de 5 à 8 chainons, de préférence saturé, est choisi parmi pipéridyle, pyrrolidinyle, pipérazinyle, morpholinyle ;
▪ les radicaux « *aryle* » ou « *hétéroaryle* » ou la partie aryle ou *hétéroaryle* d'un radical peuvent être substitués par au moins un substituant porté par un atome de carbone, choisi parmi :
   - un radical alkyle en C₁-C₆, éventuellement substitué;
   - un atome d'halogène;
   - un groupement hydroxyle ;
   - un radical alkoxy en C₁-C₂ ;
   - un radical (poly)-hydroxyalkoxy en C₂-C₄ ;
   - un radical amino ;
   - un radical héterocycloalkyle à 5 ou 6 chaînons ;
   - un radical hétéroaryle à 5 ou 6 chaînons éventuellement substitué par un radical (C₁-C₄) alkyle, préférentiellement méthyle ;
   - un radical amino substitué par un ou deux radicaux alkyle, identiques ou différents, en C₁-C₆ éventuellement porteurs d'au moins :
      i) un groupement hydroxyle,
      ii) un groupement amino éventuellement substitué par un ou deux radicaux alkyle en C₁-C₃ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - un radical acylamino (-NR-C(O)-R') dans lequel le radical R est un atome d'hydrogène, un radical alkyle en C₁-C₄ éventuellement porteur d'au moins un groupement hydroxyle et le radical R' est un radical alkyle en C₁-C₂ ;
   - un radical carbamoyle ((R)₂N-C(O)-) dans lequel les radicaux R, identiques ou non, représentent un atome d'hydrogène, un radical alkyle en C₁-C₄ éventuellement porteur d'au moins un groupement hydroxyle ;
   - un radical alkylsulfonylamino (R'-S(O)₂-N(R)-) dans lequel le radical R représente un atome d'hydrogène, un radical alkyle en C₁-C₄ éventuellement porteur d'au moins un groupement hydroxyle et le radical R' représente un radical alkyle en C₁-C₄, un radical phényle ; un radical aminosulfonyle ((R)₂N-S(O)₂-) dans lequel les radicaux R, identiques ou non, représentent un atome d'hydrogène, un radical alkyle en C₁-C₄ éventuellement porteur d'au moins un groupement hydroxyle ;
   - un radical carboxylique sous forme acide ou salifiée (de préférence avec un métal alcalin ou un ammonium, substitué ou non) ;
   - un groupement cyano ;
   - un groupement nitro ou nitroso ;
   - un groupement polyhalogénoalkyle, préférentiellement le trifluorométhyle ; par « *alkyle en C₁-C₆, éventuellement substitué* » on entend que l'alkyle peut être substitué par un ou plusieurs radicaux choisis parmi les radicaux hydroxyle, alkoxy en C₁-C₂, (poly)-hydroxyalkoxy en C₂-C₄, acylamino, amino substitué par deux radicaux alkyle, identiques ou différents, en C₁-C₄ , éventuellement porteurs d'au moins un groupement hydroxyle ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
▪ l'expression « *au moins un* » est équivalente à « *un ou plusieurs* » ; et
▪ l'expression « *inclusivement* » pour une gamme de concentrations signifie que les bornes de la gamme font partie de l'intervalle défini.

### a) Les polysaccharides aminés et oxydés à groupe(s) carboxyl(late)

Le ou les polysaccharides de l'invention sont aminés, i.e. qu'ils comprennent au moins un groupe amino. Par « *à groupe(s) amino* » on entend que le polysaccharide est substitué par un ou plusieurs groupe(s) amino NR₁R₂ i.e. au moins un des groupes hydroxy de l'unité osidique est remplacé par un groupe NR₁R₂, avec R₁ et R₂ représentant un atome d'hydrogène.

En outre le ou les polysaccharides de l'invention sont oxydés, et ils comprennent au moins un groupe carboxy(late) cela sous-entend qu'ils comprennent au moins un groupe carboxy -C(O)-OH ou carboxylate -C(O)O⁻, M⁺ avec M⁺ représentant un contre ion cationique. De préférence M⁺ représente un métal alcalin, ou alcalino-terreux tel que Na⁺, K⁺ ou un ammonium R,R',R"N⁺- avec R, R' et R", identiques ou différents, représentent un atome d'hydrogène, un groupe (C₁-C₆)alkyle éventuellement substitué notamment par un groupe hydroxy tel que hydroxyéthyle .

Selon un mode de réalisation particulier de l'invention, le ou les polysaccharide(s) aminé(s) et oxydé(s) comprenant au moins un groupe carboxylate ou acide carboxylique est(sont) anioniques ou non ioniques.

Par « *polysaccharides* » on entend les glycanes, polyosides, polyholosides ou glucides complexes, qui sont des polymères constitués de plusieurs oses liés entre eux par des liaisons O-osidiques. Il s'agit plus particulièrement de polymères formés d'un certain nombre d'oses (ou monosaccharides) ayant pour formule générale : -[Cₓ(H₂O)_{y})]ₙ- avec x un entier supérieur ou égal à 5, de préférence x est supérieur ou égal à 6, en particulier x est compris inclusivement entre 5 et 7, de préférence x = 6, et y un entier qui représente x - 1, et n est un entier supérieur ou égal à 2, particulièrement compris inclusivement entre 3 et 1000, plus particulièrement entre 5 et 500, préférentiellement entre 10 et 200

Selon un mode de réalisation particulier de l'invention, les polysaccharides aminés et oxydés à groupe(s) carboxyl(ates), anioniques ou non ioniques, sont constitués d'unités monosaccharides pouvant comprendre cinq atomes de carbone ou plus, de préférence six atomes de carbone ou plus, et plus particulièrement six atomes de carbone.

Les polysaccharides aminés et oxydés non ioniques ou anioniques comprennent en outre un ou plusieurs aldéhydes et éventuellement un ou plusieurs groupes anioniques.

Les polysaccharides aminés et oxydés comprenant au moins un groupe carboxy(late), selon l'invention sont choisis parmi ceux de formule **(I)** suivante :

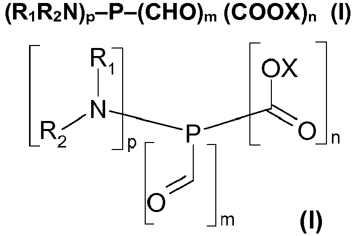

Correspondant à Formule **(I)** dans laquelle :
- **P** représente une chaîne polysaccharidique constituée de monosaccharides comprenant 5 atomes de carbone ou plus de 5 atomes de carbone, de préférence 6 ou plus de 6 atomes de carbone et plus particulièrement 6 atomes de carbone ;
- **R₁** et **R₂,** représentent un atome d'hydrogène;
- X représente atome d'hydrogène, un contre ion cationique, en particulier choisi parmi i) métal alcalin, ii) alcalino terreux tels que sodium, potassium, iii) ammonium R,R',R"N⁺ avec R, R' et R" tels que définis précédemment, et iv) les contres ions cationiques issus de base organiques telles que l'ammoniaque, les amines organiques comme la monoéthanolamine, la diéthanolamine, la triéthanolamine et l'amino-3 propanediol-1,2 et les aminoacides basiques tels que la lysine, l'arginine, la sarcosine, l'ornithine et la citrulline ;
- **m + n** est supérieur ou égal à 1 ;
- **m** est tel que le degré de substitution du polysaccharide par un ou plusieurs groupements aldéhydes (DS(CHO)), qui est supérieur ou égal à 0, en particulier m est compris dans l'intervalle allant de 0 à 2, plus particulièrement allant de 0,001 à 1,8, de préférence allant de 0,005 à 1,5 ;
- **n** est tel que le degré de substitution du polysaccharide par un ou plusieurs groupements carboxyliques (DS(COOX)), est supérieur à 0, en particulier inférieur ou égale à 2, de préférence compris inclusivement entre 0,001 et 1,5
- **p** est tel que le degré de substitution du polysaccharide par un ou plusieurs groupements amines (DS(R₁R₂N)), est supérieur à 0, en particulier inférieur ou égale à 2, de préférence compris inclusivement entre 0,001 et 1,5.

Par degré de substitution DS(CHO) ou DS (COOX) des polysaccharides selon l'invention, on entend le rapport entre le nombre de carbones oxydés en un groupement aldéhyde ou carboxylique pour tous les motifs répétitifs et le nombre de monosaccharides élémentaires (même ouverts par préoxydation) constituant le polysaccharide.

Par degré de substitution (DS(R₁R₂N)), des polysaccharides selon l'invention, on entend le rapport entre le nombre de groupe hydroxy substitué par un groupe amine NR₁R₂ pour tous les motifs répétitifs et le nombre de monosaccharides élémentaires (même ouverts) constituant le polysaccharide.

Les groupes aldéhydes CHO et carboxy(lates) COOX peuvent être obtenus lors de l'oxydation de certains atomes de carbone, par exemple en position C2, C3 ou C6, d'un motif saccharidique comprenant 6 atomes de carbone ; De préférence, l'oxydation se fait en C2 et en C3, plus particulièrement de 0,01% à 75% en nombre, et de préférence de 0,1% à 50% en nombre des cycles pouvant avoir été ouverts.

Les groupes amines R₁R₂N peuvent être obtenus lors de l'amination réductrice de certains groupes hydroxy portés par les atomes de carbone, par exemple en position C2, C3 ou C6, d'un motif saccharidique comprenant 6 atomes de carbone ; De préférence, l'amination réductrice se fait en C2 et en C3, plus particulièrement en C2 de 0,01 % à 75 % en nombre, et de préférence de 0,1 % à 50 % en nombre des cycles pouvant avoir été ouverts.

La chaîne polysaccharidique, représentée par **P** dans la formule (I) précédente, est de préférence choisie parmi les inulines, les celluloses, les amidons, les gommes de guar, les gommes de xanthane, les gommes de pullulane, les gommes alginate, les gommes d'agar-agar, les gommes de carragheenane, les gommes de gellane, le chitosan, les gommes arabique, les xyloses et les gommes adragante et leurs dérivés, le cellobiose, la maltodextrine, le scléroglucane, le chitosane, l'ulvane, le fucoïdane, l'alginate, la pectine, l'héparine, l'acide hyaluronique ou leurs mélanges.

Selon un mode particulier de l'invention la chaîne polysaccharidique, le ou les polysaccharides aminés et oxydés comprenant au moins un groupe carboxy(late), selon l'invention sont choisis parmi ceux de formule **(I)** telle que définie précédemment dans laquelle **P** correspondant à la chaîne polysaccharidique est choisie parmi les celluloses, les (C₁-C₆)alkylcelluloses, les hydroxy(C₁-C₆)alkylceliuloses telles que les hydroxyéthylcelluloses et les hydroxypropylcelluloses, les carboxycellulose et carboxy(C₁-C₆)alkylcelluloses telles que les carboxyméthylcelluloses, les amidons, et les inulines, et de préférence l'inuline.

Plus préférentiellement, la chaîne polysaccharidique est choisie parmi les inulines ou les amidons.

Plus préférentiellement encore, la chaîne polysaccharidique est l'inuline.

Par dérivé, on entend les composés obtenus par modification chimique des composés cités. Il peut s'agir d'esters, d'amides, d'éthers desdits composés.

L'oxydation peut se faire selon un procédé connu dans la technique, par exemple selon le procédé décrit dans FR 2 842 200, dans le document FR2854161 ou dans l'article "Hydrophobic films from maize bran hemicelluloses" de E. Fredon et al, Carbohydrate Polymers 49, 2002, pages 1 à 12. Un autre procédé d'oxydation est décrit dans l'article « water soluble oxidized starches by peroxide reaction extrusion » Industril Crops and Products 75 (1997) 45-52 - R. E. Wing, J. L. Willet. Ces procédés d'oxydation sont simples à mettre en oeuvre, sont efficaces, ne génèrent pas de sous-produits toxiques ou difficiles à éliminer.

Les peroxydes pouvant être utilisés au cours de ces procédés d'oxydation peuvent être un percarbonate ou un perborate de métal alcalin ou de métal alcalino-terreux, un peroxyde d'alkyle, l'acide peracétique ou le peroxyde d'hydrogène. Le peroxyde d'hydrogène est particulièrement préféré, dans la mesure où il est aisément accessible et qu'il ne produit pas de sous-produits gênants.

La quantité de peroxyde dans le milieu réactionnel est entre 0,05 et 1 équivalent molaire par unité glucose du polysaccharide, de préférence entre 0,1 et 0,8 équivalent molaire. Il est préférable d'ajouter le peroxyde par fractions successives, en laissant le milieu réactionnel sous agitation entre deux additions.

Au cours du procédé d'oxydation, on peut utiliser comme catalyseur une phtalocyanine unique ou un mélange de phtalocyanines, par exemple un mélange de phtalocyanine de Co et de phtalocyanine de Fe. La quantité de catalyseur dépend du degré de substitution souhaité. En général une faible quantité, par exemple une quantité correspondant à 0,003 à 0,016 équivalent molaire pour 100 unités glucose de polysaccharide est convenable.

Le procédé peut également être mis en oeuvre en mettant en contact le polysaccharide à l'état pulvérulent avec le catalyseur dissous dans un faible volume d'eau et avec le peroxyde. Ce procédé est désigné par procédé « demi-sec ».

Le procédé peut être mis en oeuvre par extrusion réactive en présence de peroxyde.

Pour l'amination réductrice, il est préféré d'utiliser en premier un polysaccharide oxydé au préalable selon les techniques connues de l'homme du métier telles que cités précédemment, puis de le faire réagir avec NH₃ en particulier NH₃ aqueux ou l'ammoniaque. La réaction est suivie d'une étape de réduction qui peut être réalisée avec un catalyseur tel que Ni, ou Pd sur graphite en présence de H₂ sous pression (tel que 10 bars) (voir par exemple US 6,596,861 ou Chem. Eur. J. 1130-1144 (2007) ; ibid 10196-10200 (2008)).

Préférentiellement dans le procédé de préparation du ou des polysaccharides a) de l'invention, le polysaccharide est d'abord oxydé comme décrit précédemment suivi d'une ou plusieurs aminations réductrices successives.

Plus préférentiellement, le ou les polysaccharides a) selon l'invention sont obtenus par oxydation et amination réductrice:
- d'inuline,
- des celluloses en particulier i) la cellulose, ii) les carboxy(C₁-C₆)alkylceliuloses telles que carboxyméthylcellulose, iii) les (hydroxy)(C₁-C₆)alkylceliuloses telles que les méthylcellulose, hydroxyéthylcellulose, hydroxypropylcellulose, hydroxypropylméthylcellulose,
- des amidons, en particulier i) l'amidon, ii) les acétate d'amidon, iii) les (hydroxy)(C₁-C₆)alkylamidon tels que les hydroxy-éthylamidon, hydroxypropylamidon,
- des gommes de guar, gommes de carboxy(C₁-C₆)alkylguar telles que carboxyméthylguar, gommes de carboxy(C₁-C₆)alkylhydroxy(C₁-C₆)alkylguar telles que les gommes de carboxyméthylhydroxypropylguar, gommes d'hydroxy(C₁-C₆)alkylguar, les gommes d'hydroxyéthylguar, les gommes d'hydroxypropylguar,
- des xyloses ou gommes de xanthane, gommes de carraghenane, de cellobiose, de maltodextrine, de scléroglucane, de chitosane, d'ulvane, de fucoïdane, d'alginate, de pectine, d'héparine et d'acide hyaluronique
- La cyclodextrine et ses dérivés
- ou leurs mélanges.

Préférentiellement, le polysaccharide est obtenu par oxydation et amination reductrice d'inuline ou d'amidon, plus préférentiellement par oxydation et amination reductricede l'inuline.

Préférentiellement, le polysaccharide est obtenu par oxydation et amination reductrice d'inuline.

Selon un mode de réalisation, le polysaccharide est obtenu par oxydation d'inuline en mettant en oeuvre un procédé d'extrusion réactive en présence de peroxyde d'hydrogène.

La chaîne polysaccharidique avant et après oxydation et amination réductrice présente de préférence une masse moléculaire moyenne en poids allant de 400 à 15 000000, mieux encore de 500 à 10 000000 et plus particulièrement de 500 à 50000 g/mol.

Les polysaccharides les plus particulièrement préférés dans l'invention sont ceux répondant à la formule **(I)** dans laquelle : **P** représente une chaîne polymérique issue d'inuline ou d'amidon, de préférence inuline ; **m** est tel que le degré de substitution du polysaccharide par un ou plusieurs groupements aldéhydes (DS(CHO)), est supérieur ou égal à 0 et inférieur à à 2,5, **n** est tel que le degré de substitution du polysaccharide par un ou plusieurs groupements carboxyliques (DS(COOX)), est compris dans l'intervalle allant de 0,001 à 2, et **p** est tel que le degré de substitution du polysaccharide par un ou plusieurs groupements amines (DS(R₁R₂N)), est compris inclusivement entre 0,001 et 2.

De façon encore plus préféré, le radical **P** de la formule **(I)** telle que définie précédemment représente une chaine polymérique issue d'inuline, **m** est compris dans l'intervalle allant de 0 à 1, **n** et **p,** identiques ou différents sont compris dans l'intervalle allant de 0,01 à 2.

Avantageusement, le ou les polysaccharide(s) aminé(s) et oxydé(s) tel(s) que défini(s) précédemment se trouve(nt) en une teneur allant de 0,05 à 15 % en poids, par rapport au poids total de la composition, de préférence allant de 0,1 à 10 % en poids, plus préférentiellement allant de 0,2 à 6 % en poids.

### les compositions

La composition de l'invention comprend un ou plusieurs polysaccharide(s) aminé(s) oxydé(s) comprenant au moins un groupe carboxy(late) tel(s) que défini(s) précédemment.

La ou les compositions de l'invention sont cosmétiques i.e. contiennent un milieu physiologiquement acceptable, c'est-à-dire compatible avec les matières kératiniques d'êtres humains tels que la peau (corps, visage, contour des yeux, cuir chevelu), les cheveux, les cils, les sourcils, les poils, les ongles, les lèvres.

Le milieu physiologiquement acceptable de la ou des compositions mises en oeuvre dans le procédé selon l'invention est avantageusement un milieu aqueux. Il peut par exemple être constitué par de l'eau ou par un mélange d'eau et d'au moins un solvant organique cosmétiquement acceptable. A titre de solvant organique, on peut par exemple citer les alcools inférieurs en C₂-C₄, tels que l'éthanol et l'isopropanol ; les polyols , notamment ceux ayant de 2 à 6 atomes de carbones comme la glycérine, le propylène glycol, le butylène glycol, le pentylène glycol, l'hexylène glycol, le dipropylène glycol, le diéthylène glycol ; les éthers de polyols comme le 2-butoxyéthanol, le monométhyléther de propylèneglycol, le monoéthyléther et le monométhyléther du diéthylèneglycol ; et leurs mélanges.

De préférence, la composition cosmétique comprend de 50 à 99,5% en poids d'eau par rapport au poids de la composition.

La composition utilisée selon l'invention peut contenir en outre un ou plusieurs additifs cosmétiques choisi parmi les agents tensio-actifs non-ioniques, anioniques, cationiques et amphotères, les vitamines et pro-vitamines dont le panthénol, les filtres solaires, les charges, les matières colorantes, les agents nacrants, les agents opacifiants, les agents séquestrants, les polymères filmogènes, les polymères différents des polysaccharides a) selon l'invention, cationiques, anioniques ou neutres, les polymères associatifs, les agents plastifiants, les agents épaississants, les huiles, les agents anti-oxydants, les agents anti-mousse, les agents hydratants, les agents émollients, les agents de pénétration, les parfums et les agents conservateurs ; de préférence un ou plusieurs tensioactifs non-ioniques, anioniques, cationiques et amphotères, polymères différents des polysaccharides a) selon l'invention, cationiques, anioniques, neutres, ou polymères associatifs.

La composition utilisée selon l'invention peut se présenter sous toutes les formes galéniques classiquement utilisées pour une application capillaire et notamment sous forme de solutions aqueuses, solutions hydroalcooliques, d'émulsions huile-dans-eau (H/E) ou eau-dans-huile (E/H) ou multiple (triple : E/H/E ou H/E/H), de gels aqueux, de gels hydroalcooliques. Ces compositions sont préparées selon les méthodes usuelles. De préférence, la composition est sous forme de solution ou de gel aqueux ou hydroalcoolique.

### pH de la ou des compositions :

Selon un mode de réalisation particulier de l'invention la composition qui comprend a) un ou plusieurs polysaccharide(s) aminé(s) oxydé(s) comprenant au moins un groupe carboxy(late) tel(s) que défini(s) précédemment notamment l'inuline, se trouve à un pH compris inclusivement entre 2,5 et 9,5.

Selon un mode de réalisation préféré de l'invention la composition qui comprend l'ingrédient a) de l'invention est une composition aqueuse qui se trouve à un pH acide, en particulier à un pH compris inclusivement entre 1 et 6, plus particulièrement entre 2 et 5, de préférence entre 3 et 4.

Les pH peuvent être ajustés par un acide organique ou minéral, ou par un agent alcalin choisi parmi les agents alcalins minéraux ou organiques ou hybrides ou leurs mélanges.

Par « *acide organique* » on entend un acide i.e. un composé capable de libérer un cation ou proton H⁺, ou H₃O⁺ en milieux aqueux, qui comporte au moins une chaine hydrocarbonée en C₁-C₂₀, éventuellement insaturée, linéaire ou ramifiée, un groupe (hétéro)cycloalkyle, ou (hétéro)aryle et au moins une fonction chimique acide étant en particulier choisie parmi carboxy C(O)OH, sulfurique SO₃H, SO₂H, et phosphorique PO₃H₂, PO₄H₂.

Plus particulièrement les acides utilisés sont choisis parmi l'acide chlorhydrique HCl, d'acide bromhydrique HBr, l'acide sulfurique H₂SO₄, acides alkylsulfoniques : (C₁-C₆)Alk-S(O)₂OH tels que d'acide méthylsulfonique et d'acide éthylsulfonique ; acides arylsulfoniques : Ar-S(O)₂OH tel que d'acide benzène sulfonique et d'acide toluène sulfonique ; acides (C₁-C₆)alkoxysulfiniques : Alk-O-S(O)OH tels que acide méthoxysulfinique et acide éthoxysulfinique ; acides aryloxysulfiniques tels que d'acide toluèneoxysulfinique et acide phénoxysulfinique ; acide phosphorique H₃PO₄; acide triflique CF₃SO₃H et acide tétrafluoroborique HBF₄ et les acide(s) carboxylique(s) de formule **(II)** suivante

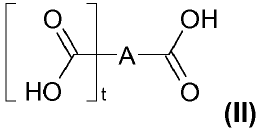

Formule **(II)** ou un de ses sels dans laquelle :
**A** représente un groupe monovalent lorsque t vaut 0 ou polyvalent lorsque t est supérieur ou égale à 1, hydrocarboné comprenant de 1 à 50 atomes de carbone, saturé ou insaturé, cyclique ou non cyclique, aromatique ou non aromatique, éventuellement interrompu par un ou plusieurs hétéroatomes et/ou éventuellement substitué notamment par un ou plusieurs groupes hydroxy; de préférence A représente un groupe monovalent (C₁-C₆)alkyle ou polyvalent (C₁-C₆)alkylène éventuellement substitué par un ou plusieurs groupes hydroxy.

Particulièrement les acides carboxylique(s) de formule (II) telle que définie précédemment et de préférence le ou les acides utilisés est ou sont un alpha hydroxyacide tels que les acides lactiques, glycoliques, tartriques ou citriques.

Le ou les agents alcalins minéraux sont de préférence choisis parmi l'ammoniaque, les carbonates ou bicarbonates alcalins comme les carbonates de sodium ou de potassium et les bicarbonates de sodium ou de potassium, les hydroxydes de sodium ou de potassium ou leurs mélanges.

Selon un mode de réalisation avantageux de l'invention le ou les agents alcalins sont des amines organiques *i.e* ils contiennent au moins un groupe amino substitué ou non..

Le ou les agents alcalins organiques sont plus préférentiellement choisis parmi les amines organiques dont le pK_{b} à 25 °C est inférieur à 12, et de préférence inférieur à 10, encore plus avantageusement inférieur à 6. Il est à noter qu'il s'agit du pK_{b} correspondant à la fonction de basicité la plus élevée.

A titre de composés hybrides on peut mentionner les sels des amines citées précédemment avec des acides comme l'acide carbonique, l'acide chlorhydrique.

Le ou les agents alcalins organiques sont par exemple choisis parmi les alcanolamines, les éthylènediamines oxyéthylénées et/ou oxypropylénées, les acides aminés et les composés de formule **(III)** suivante :

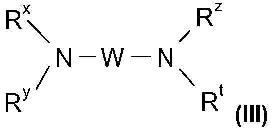

Formule **(III)** dans laquelle :
- **W** est un radical divalent alkylène en C₁-C₆ éventuellement substitué par un groupement hydroxyle ou un radical alkyle en C₁-C₆, et/ou éventuellement interrompu par un ou plusieurs hétéroatomes tels que l'oxygéne ou NR^{u} ;
- **R^{x}**, **R^{y}**, **R^{z} R^{t}** et **R^{u}**, identiques ou différents, représentent un atome d'hydrogène, un radical alkyle en C₁-C₆ ou hydroxyalkyle en C₁-C₆, aminoalkyle en C₁-C₆.

De préférence, l'alcanolamine est l'éthanolamine (ou monoéthanolamine).

Dans une variante de l'invention, la composition comprend en tant qu'agent alcalin une ou plusieurs alcanolamines (de préférence l'éthanolamine) et de l'ammoniaque. Dans cette variante, la ou les alcanolamines sont présentes en quantité majoritaire par rapport à l'ammoniaque.

Plus préférentiellement le pH est ajusté pour que le pH soit compris inclusivement entre 2,5 et 9,5 ou pH compris inclusivement entre 1 et 6, plus particulièrement entre 2 et 5, de préférence entre 3 et 4 à l'aide de NH₄OH ou de tampon citrate.

### le procédé de traitement des matières kératiniques,

Le procédé de traitement des matières kératiniques en particulier des fibres kératiniques, plus particulièrement des fibres kératiniques humaines telles que les cheveux, consiste lors d'une première étape d'appliquer sur lesdites matières une composition comprenant un ou plusieurs ingrédients a) tels que définis précédemment.

Selon un mode de réalisation particulier de l'invention le procédé est réalisé à température ambiante (25 °C) sans traitement thermique desdites matières kératiniques.

Selon un autre mode de réalisation particulier de l'invention le procédé de traitement des matières kératiniques comprend après l'application de la composition comprenant a) ; une étape supplémentaire de chauffage des matières kératiniques, notamment des fibres kératiniques de préférence à une température d'au moins 100°C, en particulier à une température comprise inclusivement entre 100 et 250°C. De préférence, l'étape de chauffage des fibres kératiniques est réalisée à une température allant de 150 à 220°C, de préférence allant de 160°C à 220°C, préférentiellement allant de 160°C à 200°C, notamment allant de 170°C à 190°C. Il est entendu que les températures de chauffages sont les températures des moyens de chauffages en particulier des plaques de fer lorsqu'il s'agit de fer, et non de la température desdites matières kératiniques.

Cette étape b) de chauffage est avantageusement réalisée au moyen d'un fer.

L'étape de chauffage permet d'optimiser les effets du procédé, notamment d'optimiser la rémanence des propriétés cosmétiques après un ou plusieurs shampooings.

Par « *fer* », on entend, au sens de la présente invention, un dispositif de chauffage des fibres kératiniques mettant en contact lesdites fibres et le dispositif de chauffage.

L'extrémité du fer venant en contact avec les matières kératiniques, notamment des fibres kératiniques, présente généralement deux surfaces planes. Ces deux surfaces peuvent être métalliques ou en céramique. En particulier, ces deux surfaces peuvent être lisses ou crantées ou courbes.

L'étape de chauffage peut être effectuée au moyen d'un fer à lisser, d'un fer à friser, d'un fer à cranter ou d'un fer vapeur. De préférence, l'étape de chauffage est effectuée au moyen d'un fer à lisser.

A titre d'exemple de fers utilisables dans le procédé de lissage selon l'invention, on peut citer tous types de fer plats, et en particulier, de manière non limitative, ceux décrits dans les brevets US 5,957,140 et US 5,046,516.

L'application du fer peut être effectuée par touches séparées successives de quelques secondes, ou par déplacement ou glissement progressif le long des mèches de fibres kératiniques, notamment de cheveux.

De préférence, l'application du fer dans le procédé selon l'invention se fait en mouvement continu de la racine à la pointe des cheveux, en un ou plusieurs passages, en particulier en deux à vingt passages. La durée de chaque passage du fer peut aller de 2 secondes à 1 minute.

De préférence, l'étape de chauffage des fibres kératiniques est effectuée pendant une durée pouvant aller de 2 secondes à 30 minutes, et préférentiellement de 2 secondes à 20 minutes , mieux de 2 secondes à 10 minutes, mieux de 2 secondes à 5 minutes, et encore mieux de 2 secondes à 2 minutes.

Le procédé selon l'invention peut également comprendre une étape supplémentaire c) de séchage des fibres kératiniques après l'application a) du ou des polysaccharide(s) aminé(s) et oxydé(s) et avant l'étape b) de chauffage des fibres kératiniques effectuée à une température d'au moins 100°C, si étape de chauffage il y a.

L'étape de séchage peut être réalisée au moyen d'un sèche-cheveux, d'un casque ou encore par séchage libre. L'étape de séchage est avantageusement effectuée à une température allant de 20 à 70°C.

Après l'étape d'application des ingrédients a) ou de chauffage b), les fibres kératiniques peuvent être éventuellement rincées à l'eau ou lavées avec un shampooing. Les fibres kératiniques sont ensuite éventuellement séchées au moyen d'un sèche-cheveux ou d'un casque ou à l'air libre.

Selon un mode de réalisation, le procédé selon l'invention est mis en oeuvre sur des fibres kératiniques naturelles, notamment des cheveux naturels.

Selon un autre mode de réalisation, le procédé selon l'invention est mis en oeuvre sur des fibres kératiniques, notamment des cheveux, abimées. Comme indiqué précédemment, on entend par cheveux abimés des cheveux secs ou rêches ou cassants ou fourchus ou mous.

Selon un autre mode de réalisation, le procédé de traitement selon l'invention est de préférence mis en oeuvre sur des fibres kératiniques, notamment des cheveux, sensibilisées, telles que des fibres décolorées, colorées artificiellement, défrisées ou permanentées.

Le procédé selon l'invention peut être mise en oeuvre sur des fibres kératiniques, notamment des cheveux, sèches ou humides. Préférentiellement, le procédé est mis en oeuvre sur fibres kératiniques sèches, notamment des cheveux secs.

La ou les compositions utilisées selon l'invention sont avantageusement appliquées sur les fibres kératiniques en une quantité allant de 0,1 à 10 grammes, de préférence de 0,2 à 5 grammes de composition par gramme de fibres kératiniques.

Après application de la composition sur les matières kératiniques, en particulier les fibres kératiniques, ces dernières peuvent être essorées pour retirer l'excès de composition ou bien lavées à l'eau ou avec un shampooing.

Après application sur les matières kératiniques, en particulier les fibres kératiniques des ingrédients a) tels que définis précédemment, et avant d'effectuer b) l'étape de chauffage desdites matières kératiniques, si étape de chauffage il y a, selon un mode particulier de l'invention a) on laisse poser le(s) polysaccharide(s) aminé(s) et oxydé(s) a) tel(s) que défini(s) précédemment pendant une durée allant de 1 à 60 minutes, de préférence allant de 2 à 50 minutes, préférentiellement allant de 5 à 45 minutes. Le temps de pose peut être effectué à une température allant de 15°C à 45°C, de préférence à la température ambiante (25°C).

Selon un mode de réalisation du procédé selon l'invention, le ou les polysaccharide(s) aminé(s) et oxydé(s) comprenant au moins un groupe carboxy(late) a) tel(s) que défini(s) précédemment et le ou les (poly)saccharides à groupe(s) amine(s) b) tel(s) que défini(s) précédemment ; a) et b) sont présents dans des compositions distinctes. Ils sont donc appliqués séparément sur lesdites matières kératiniques.

Selon un mode particulier du procédé de de l'invention l'étape d'application du ou des polysaccharide(s) oxydé(s) comprenant au moins un groupe carboxy(late) a) met en oeuvre une composition comprenant au moins un polysaccharide aminé et oxydé comprenant au moins un groupe carboxy(late) tel que défini précédemment, en une teneur allant de 0,05 à 15 % en poids, par rapport au poids total de la composition, de préférence allant de 0,1 à 10 % en poids, plus préférentiellement allant de 0,2 à 6 % en poids.

Selon un mode de réalisation du procédé selon l'invention, on effectue dans l'ordre suivant : l'étape d'application de l'ingrédient a) puis l'étape de chauffage b). Avantageusement, les ingrédients a) sont présents dans une composition cosmétique.

Le procédé de traitement selon l'invention peut être mis en oeuvre avant, pendant et/ou après un procédé de traitement cosmétique additionnel des fibres kératiniques, tels qu'un procédé de mise en forme temporaire (mise en forme avec bigoudis, fer à friser, fer à lisser) ou un procédé de mise en forme durable (permanente, défrisage) des fibres kératiniques.

Le procédé de traitement peut être mis en oeuvre en pré-traitement d'un procédé de coloration, de défrisage et/ou d'un procédé de permanente afin de protéger cosmétiquement les fibres kératiniques de ces traitements. En d'autres termes, ce procédé est effectué pour préserver les propriétés cosmétiques des fibres kératiniques avant un procédé de traitement cosmétique tel que décrit précédemment.

En particulier, le procédé de traitement est mis en oeuvre en post-traitement d'un procédé de décoloration, de coloration artificielle, de défrisage et/ou d'un procédé de permanente afin de réparer lesdites fibres.

Le procédé selon l'invention peut être mis en œuvre pendant un procédé de traitement cosmétique afin de réparer lesdites fibres.

En particulier, le procédé de traitement selon l'invention peut être mis en oeuvre sur des fibres kératiniques abîmées.

Autrement dit, le procédé de traitement selon l'invention est de préférence mis en oeuvre sur des fibres kératiniques sensibilisées telles que des fibres décolorées, colorées, défrisées ou permanentées.

En particulier, le procédé de traitement peut être mis en oeuvre avant un procédé de décoloration, de coloration, de défrisage et/ou d'un procédé de permanente des fibres kératiniques.

En variante, le procédé de traitement peut être mis en oeuvre pendant et/ou après un procédé de traitement cosmétique des fibres kératiniques, en particulier :
i) pendant et/ou après un procédé de coloration ou un procédé de permanente ou un procédé de défrisage des fibres kératiniques, et
ii) après un procédé de décoloration des fibres kératiniques.

Selon un mode de réalisation, le procédé de traitement selon l'invention est mis en oeuvre après un procédé de décoloration des fibres kératiniques.

### Le kit

L'invention a également pour objet un kit ou dispositif à plusieurs compartiments comprenant :
- une première composition comprenant a) au moins un polysaccharide aminé et oxydé comprenant au moins un groupe carboxy(late) tel que défini précédemment ; et
- éventuellement un dispositif pour chauffer les fibres kératiniques à une température d'au moins 100 °C, de préférence allant de 100 à 250 °C.

L'ensemble de conditionnement des compositions est de façon connue tout packaging adapté pour stocker les compositions cosmétiques (flacons, tube, flacon spray, flacon aérosol notamment).

Un tel kit permet de mettre en oeuvre le procédé de traitement des fibres kératiniques selon l'invention.

Les exemples suivants sont donnés à titre illustratif de la présente invention.

Les quantités indiquées dans les exemples sont exprimées en pourcentage en poids.

### EXEMPLES

Les compositions suivantes ont été préparées, les % sont des pourcentages massiques en g pour 100 g de composition

| Compositions | | Ingrédients |
|---|---|---|
| Composition 1 | Témoin | Eau MilliQ uniquement |
| Composition 2 | Invention | Inuline aminée, oxydée et carboxylée* à 5% dans l'eau, pH spontané 5,5 |
| Composition 3 | Comparatif | Inuline oxydée** à 5% dans l'eau , pH spontané 3 |

| | | |
|---|---|---|
| ** *L'Inuline oxydé (IO) été préparé par oxydation d'inuline commercialisée sous la dénomination « INUTEC N25 » par la société ORAFTI, en mettant en œuvre un procédé d'extrusion réactive tel que décrit dans l'article "*Water soluble oxidized starches by peroxide reactive extrusion" de R.E. Wing et J.L. Willett, Industrial Crops and Products 7, 1997, pages 45-52*. On a utilisé une extrudeuse bivis co-rotative de type BC21 commercialisée par la Société Clextral et l'eau oxygénée comme agent oxydant. IO est donc obtenue par extrusion réactive d'un mélange de 78 % en poids d'inuline et de 1,57 % en poids d'eau oxygénée dans l'eau ; le pH spontané après extrusion réactive est de 3,8. L'IO ainsi obtenu a une teneur en carbonyle de 1,23 % (p*/*p) et une teneur en carboxyle de 0,17 % (plp).* * *l'inuline aminée oxydée et carboxylée selon invention est obtenue à partir de l'IO qui a été aminée avec de l'ammoniaque, réduit avec du palladium sur graphite en présence d'hydrogène sous pression (20 bars) chauffé à 50 °C plusieurs heures (entre 10 h. et 24 h), suivie d'élimination du Pd*/*C, d'azote et de l'eau, l'étape d'amination a été réalisée selon la technologie classique d'amination réductrice sous forme de sel d'ammonium d'inuline à partir (cf.* Chem. Eur. J., 1130-1144 (2007*), ibid 14, 10196 (2008)).* *Les analyses physicochimiques sont en accord avec la présence de groupes amino, carbonyles et carboxy dans l'inuline de l'invention.* | | |

### Traitement

### Protocole d'évaluation

Les essais d'évaluation de traitement des fibres kératiniques ont été réalisés de la manière suivante :
Des mèches de 20 cm et 2,7 g de cheveux caucasiens raides ont été décolorées avec 2 déco INFINIE PLATINE, rincées et séchées puis des mèches de cheveux secs ont été mises en contact avec une des compositions 1 à 3 à raison de 2 g de composition pour 1 g de cheveu, 30 minutes d'immersion à 55 °C ; suivi :
- d'essorage des mèches, puis séchage au sèche-cheveu 60 °C avec passage de brosse douce ;
- d'application du fer à lisser à 210°C, 15 passages de 5 secondes ;
- du mouillage des mèches à l'eau du robinet, de l'application d'un shampooing DOP camomille à raison de 0,4 g / g de cheveux, du massage 15 secondes et rinçage soigneux à l'eau à 37°C pendant 10 secondes ; puis
- du séchage à l'étuve à 60°C pendant 10 minutes.

### Evaluations et tests réalisés

Toucher : Après application et séchage, les mèches ont été évaluées sensoriellement à sec par 5 personnes formées à l'évaluation sensorielle sur cheveu, sur des critères tactiles. Un scoring de 1 (mèches très rêches au toucher) à 5 (mèches très douces au toucher) a été attribué. Les moyennes de ces scores sont reportées.

Démêlage : Des tests de démêlage au peigne ont été réalisés après immersion de 10 secondes dans l'eau, par 5 passages de peigne plastique à petites dents (7 dents / cm, diamètre des dents env. 800 µm). Un scoring de 1 (mèches très difficiles à démêler) à 5 (mèches très faciles à démêler) a été attribué. Les moyennes de ces scores sont reportées.

Lisse visuel *:* Une évaluation du lisse visuel est réalisée sur l'aspect à sec des mèches traitées après 48 heures maintenues à 55 % HR. Un scoring de 1 (non lisse) à 5 (mèches lisses) a été attribué. Les moyennes de ces scores sont reportées.

### Résultats :

Les mèches ont été évaluées par 5 testeurs. On obtient les résultats suivants (1 : plus basse note à 5 : la plus élevée)

| Compositions testée | Démêlage humide | Démêlage sec | Lisse visuel sec (discipline) | Toucher sec |
|---|---|---|---|---|
| Composition 1 (Placébo) | 1,3 | 1,1 | 1,4 | 1,3 |
| Composition 2 (invention) | 3,9 | 3,8 | 4,7 | 3,8 |
| Composition 3 (comparatif) | 1,8 | 2,1 | 2,9 | 3,2 |

Il apparait que le procédé de traitement des matières kératiniques selon l'invention et la composition de l'invention mise en œuvre dans le procédé permet d'améliorer de façon significative le démêlage, le lissage et le toucher desdites matières.

## Revendications

1. Procédé de traitement des matières kératiniques, en particulier des fibres kératiniques, notamment humaines, de préférence des cheveux, comprenant au moins une étape consistant à appliquer sur lesdites fibres a) un ou plusieurs polysaccharide(s) aminé(s) et oxydé(s) comprenant au moins un groupe carboxy-C(O)-OH ou carboxylate-C(O)O⁻, M⁺ avec M⁺ représentant un contre ion cationique, dans lequel que le ou lesdits polysaccharidiques oxydés a) sont représentés par la formule **(I)** suivante :
**(R₁R₂N)ₚ-P-(CHO)ₘ (COOX)ₙ** **(I)**
Formule **(I)** dans laquelle :
• **P** représente une chaîne polysaccharidique constituée de monosaccharides comprenant 5 atomes de carbone ou plus de 5 atomes de carbone, de préférence 6 ou plus de 6 atomes de carbone et plus particulièrement 6 atomes de carbone ;
• **R₁** et **R₂** représentent un atome d'hydrogène ;
• **X** représente atome d'hydrogène, un contre ion cationique en particulier choisi parmi : i) métal alcalin, ii) alcalino terreux tels que sodium, ou potassium, iii) ammonium R,R',R"N⁺- avec R, R' et R", identiques ou différents, représentent un atome d'hydrogène, un groupe (C₁-C₆)alkyle éventuellement substitué notamment par un groupe hydroxy tel que hydroxyéthyle ; iv) et un contre ion cationique issu de l'ammoniaque, la monoéthanolamine, la diéthanolamine, la triéthanolamine et l'amino-3 propanediol-1,2 et les aminoacides basiques tels que la lysine, l'arginine, la sarcosine, l'ornithine et la citrulline, ;
• **m + n** est supérieur ou égal à 1 ;
• **m** est tel que le degré de substitution du polysaccharide par un ou plusieurs groupements aldéhydes (DS(CHO)), qui est supérieur ou égal à 0, en particulier m est compris dans l'intervalle allant de 0 à 2, plus particulièrement allant de 0,001 à 1,8, de préférence allant de 0,005 à 1,5 ;
• **n** est tel que le degré de substitution du polysaccharide par un ou plusieurs groupements carboxyliques (DS(COOX)), est supérieur à 0, en particulier inférieur ou égale à 2, de préférence compris inclusivement entre 0,001 et 1,5
• **p** est tel que le degré de substitution du polysaccharide par un ou plusieurs groupements amino (DS(R₁R₂N)), est supérieur à 0, en particulier inférieur ou égale à 2, de préférence compris inclusivement entre 0,001 et 1,5.

2. Procédé selon la revendication précédente, dans lequel le ou les polysaccharides a) sont non ioniques ou anioniques.

3. Procédé selon l'une quelconque des revendications précédentes, dans laquelle m est compris dans l'intervalle allant de 0 à 1, **n** et **p,** identiques ou différents sont compris dans l'intervalle allant de 0,01 à 2.

4. Procédé selon la revendication 3, **caractérisé en ce que** la chaîne polysaccharidique est choisie parmi les celluloses, les amidons, les gommes de guar, les inulines, les gommes de xanthane, les gommes de pullulane, les gommes d'agar-d'agar, les gommes de carragheenane, les gommes de gellane, les gommes arabique, les gommes adragante, les xylanes et leurs dérivés, le cellobiose, la maltodextrine, le scléroglucane, le chitosane, l'ulvane, le fucoïdane, l'alginate, la pectine, l'héparine, l'acide hyaluronique, la cyclodextrine et ses dérivés, et leurs mélanges

5. Procédé selon la revendication 3, **caractérisé en ce que** la chaîne polysaccharidique est choisie parmi les celluloses, les (C₁-C₆)alkylcelluloses, les hydroxy(C₁-C₆)alkylcelluloses telles que les hydroxyéthylcelluloses et les hydroxypropylcelluloses, les carboxycellulose et carboxy(C₁-C₆)alkylcelluloses telles que les carboxyméthylcelluloses, les amidons, et les inulines, et de préférence l'inuline.

6. Procédé selon l'une quelconque des revendications précédentes, dans lequel l'étape d'application du ou des polysaccharide(s) aminé(s) et oxydé(s) comprenant au moins un groupe carboxy-C(O)-OH ou carboxylate -C(O)O⁻, M⁺ avec M⁺ représentant un contre ion cationique met en œuvre une composition comprenant au moins un polysaccharide aminé et oxydé comprenant au moins un groupe carboxylate tel que défini dans une quelconque des revendications précédente, en une teneur allant de 0,05 à 15 % en poids, par rapport au poids total de la composition, en particulier allant de 0,1 à 10 % en poids, plus préférentiellement allant de 0,2 à 6 % en poids.

7. Procédé selon l'une quelconque des revendications précédentes, qui met en œuvre une composition comprenant au moins un polysaccharide aminé et oxydé comprenant au moins un groupe carboxylate tel que défini dans une quelconque des revendications précédentes, ladite composition étant aqueuse et à un pH compris inclusivement entre 1 et 9,5 ; en particulier ladite composition est à pH acide, en plus particulièrement compris inclusivement entre 2 et 6, encore plus particulièrement entre 2,5 et 5, de préférence entre 3 et 4.

8. Procédé selon l'une quelconque des revendications précédentes, qui met en œuvre une composition comprenant au moins un polysaccharide aminé et oxydé comprenant au moins un groupe carboxylate tel que défini dans une quelconque des revendications 1 à 6, ladite composition comprenant en outre un ou plusieurs ingrédients supplémentaires choisis parmi les tensioactifs non-ioniques, anioniques, cationiques et amphotères, vitamines et pro-vitamines dont le panthénol, filtres solaires, les charges, matières colorantes, agents nacrants, agents opacifiants, agents séquestrants, polymères filmogènes, polymères différents du ou des polysaccharides a), cationiques, anioniques ou neutres, polymères associatifs, agents plastifiants, agents épaississants, huiles, agents anti-oxydants, agents anti-mousse, agents hydratants, agents émollients, agents de pénétration, parfums et agents conservateurs ; de préférence la composition comprend en outre un ou plusieurs tensioactifs non-ioniques, anioniques, cationiques et amphotères, polymères différents des polysaccharides a), cationiques, anioniques, neutres, ou polymères associatifs.

9. Procédé selon l'une quelconque des revendications précédentes, qui comprend une étape supplémentaire de chauffage qui est réalisée à une température allant de 100 à 250°C, de préférence allant de 150 à 220 °C, préférentiellement allant de 160°C à 220°C, plus préférentiellement allant de 160 °C à 200 °C, de préférence cette étape a lieu après l'application du ou des polysaccharide(s) aminé(s) et oxydé(s) comprenant au moins un groupe carboxy(late) a) ; particulièrement l'étape de chauffage est effectuée avec un fer à lisser.

10. Composition aqueuse et à un pH compris inclusivement entre 1 et 9,5 ; en particulier ladite composition est à pH acide, en plus particulièrement compris inclusivement entre 2 et 6, encore plus particulièrement entre 2,5 et 5, de préférence entre 3 et 4 ; comprenant un ou plusieurs polysaccharide(s) aminé(s) et oxydé(s) comprenant au moins un groupe carboxy-C(O)-OH ou carboxylate -C(O)O⁻, M⁺ avec M⁺ représentant un contre ion cationique a) tel(s) que défini(s) dans une quelconque des revendications 1 à 6 et 8.

11. Utilisation d'un ou plusieurs polysaccharide(s) aminé(s) et oxydé(s) comprenant au moins un groupe carboxy-C(O)-OH ou carboxylate -C(O)O⁻, M⁺ avec M⁺ représentant un contre ion cationique a) tel(s) que défini(s) dans une quelconque des revendications 1 à 5, pour améliorer le conditionnement des fibres kératiniques, et/ou pour discipliner et/ou pour réparer les fibres kératiniques abimées, et/ou pour prévenir la casse des fibres kératiniques.

12. Utilisation d'une composition telle que définie dans la revendication 10, pour améliorer le conditionnement des fibres kératiniques, et/ou pour discipliner et/ou pour réparer les fibres kératiniques abimées, et/ou pour prévenir la casse des fibres kératiniques.

13. Kit ou dispositif à plusieurs compartiments comprenant :
• une première composition telle que définie dans la revendication 10 ; et
• éventuellement un dispositif pour chauffer les fibres kératiniques à une température d'au moins 100 °C, de préférence allant de 100 à 250 °C.

## Patentansprüche

1. Verfahren zur Behandlung von Keratinmaterialien, insbesondere Keratinfasern, insbesondere menschlichen Keratinfasern, vorzugsweise des Haars, das mindestens einen Schritt umfasst, der darin besteht, dass man auf die Fasern a) ein oder mehrere aminierte und oxidierte Polysaccharide mit mindestens einer Carboxygruppe -C(O)-OH oder Carboxylatgruppe -C(O)O⁻, M⁺, wobei M⁺ für ein kationisches Gegenion steht, aufbringt, wobei das bzw. die oxidierten Polysaccharide a) durch die folgende Formel (I) wiedergegeben werden:
(R₁R₂N)ₚ-P-(CHO)ₘ(COOX)ₙ (I)
wobei in Formel (I):
• P für eine Polysaccharidkette steht, die aus Monosacchariden mit 5 Kohlenstoffatomen oder mehr als 5 Kohlenstoffatomen, vorzugsweise 6 oder mehr als 6 Kohlenstoffatomen und insbesondere 6 Kohlenstoffatomen besteht;
• R₁ und R₂ für ein Wasserstoffatom stehen;
• X für ein Wasserstoffatom oder ein kationisches Gegenion, das insbesondere aus i) Alkalimetall, ii) Erdalkalimetall wie Natrium oder Kalium, iii) Ammonium R,R',R"N⁺, wobei R, R' und R", die gleich oder verschieden sind, für ein Wasserstoffatom oder eine (C₁-C₆) Alkylgruppe, die gegebenenfalls durch eine Hydroxygruppe substituiert ist, wie Hydroxyethyl, steht, iv) und einem kationischen Gegenion, das sich von wässrigem Ammoniak, Monoethanolamin, Diethanolamin, Triethanolamin und 3-Amino-1,2-propandiol und basischen Aminosäuren wie Lysin, Arginin, Sarkosin, Ornithin und Citrullin ableitet, ausgewählt ist, steht;
• m + n gleich oder größer als 1 ist;
• m so beschaffen ist, dass der Substitutionsgrad des Polysaccharids mit einer oder mehreren Aldehydgruppen (DS(CHO)) größer oder gleich 0 ist, insbesondere m im Bereich von 0 bis 2 liegt, spezieller im Bereich von 0,001 bis 1,8, vorzugsweise im Bereich von 0,005 bis 1,5, liegt;
• n so beschaffen ist, dass der Substitutionsgrad des Polysaccharids mit einer oder mehreren Carboxylgruppen (DS(COOX)) größer als 0, insbesondere kleiner oder gleich 2, ist und vorzugsweise zwischen 0,001 und 1,5 inklusive liegt;
• p so beschaffen ist, dass der Substitutionsgrad des Polysaccharids mit einer oder mehreren Aminogruppen (DS(R₁R₂N)) größer als 0, insbesondere kleiner oder gleich 2, ist und vorzugsweise zwischen 0,001 und 1,5 inklusive liegt.

2. Verfahren nach dem vorhergehenden Anspruch, wobei das bzw. die Polysaccharide a) nichtionisch oder anionisch sind.

3. Verfahren nach einem der vorhergehenden Ansprüche, wobei m im Bereich von 0 bis 1 liegt und n und p, die gleich oder verschieden sind, im Bereich von 0,01 bis 2 liegen.

4. Verfahren nach Anspruch 3, **dadurch gekennzeichnet, dass** die Polysaccharidkette aus Cellulosen, Stärken, Guargummen, Inulinen, Xanthangummen, Pullulangummen, Agar-Agar-Gummen, Carrageenangummen, Gellangummen, Gummi arabicum, Traganthgummen, Xylanen und Derivaten davon, Cellobiose, Maltodextrin, Scleroglucan, Chitosan, Ulvan, Fucoidan, Alginat, Pectin, Heparin, Hyaluronsäure, Cyclodextrin und Derivaten davon und Mischungen davon ausgewählt ist.

5. Verfahren nach Anspruch 3, **dadurch gekennzeichnet, dass** die Polysaccharidkette aus Cellulosen, (C₁-C₆)Alkylcellulosen, Hydroxy(C₁-C₆)alkylcellulosen wie Hydroxyethylcellulosen und Hydroxypropylcellulosen, Carboxycellulose und Carboxy(C₁-C₆)alkylcellulosen wie Carboxymethylcellulose, Stärken, Inulinen und vorzugsweise Inulin ausgewählt ist.

6. Verfahren nach einem der vorhergehenden Ansprüche, wobei bei dem Schritt des Aufbringens des bzw. der aminierten und oxidierten Polysaccharide mit mindestens einer Carboxygruppe -C(O)-OH oder Carboxylatgruppe -C(O)O⁻, M⁺, wobei M⁺ für ein kationisches Gegenion steht, eine Zusammensetzung eingesetzt wird, die mindestens ein aminiertes und oxidiertes Polysaccharid mit mindestens einer Carboxylatgruppe gemäß einem der vorhergehenden Ansprüche in einem Gehalt im Bereich von 0,05 bis 15 Gew.-%, bezogen auf das Gesamtgewicht der Zusammensetzung, insbesondere im Bereich von 0,1 bis 10 Gew.-%, weiter bevorzugt im Bereich von 0,2 bis 6 Gew.-%, umfasst.

7. Verfahren nach einem der vorhergehenden Ansprüche, bei dem eine Zusammensetzung, die mindestens ein aminiertes und oxidiertes Polysaccharid mit mindestens einer Carboxylatgruppe gemäß einem der vorhergehenden Ansprüche umfasst, eingesetzt wird, wobei die Zusammensetzung wässrig ist und bei einem pH-Wert zwischen 1 und 9,5 einschließlich vorliegt; insbesondere die Zusammensetzung bei saurem pH-Wert, spezieller zwischen 2 und 6 einschließlich, noch spezieller zwischen 2,5 und 5, vorzugsweise zwischen 3 und 4, vorliegt.

8. Verfahren nach einem der vorhergehenden Ansprüche, bei dem eine Zusammensetzung, die mindestens ein aminiertes und oxidiertes Polysaccharid mit mindestens einer Carboxylatgruppe gemäß einem der Ansprüche 1 bis 6 umfasst, eingesetzt wird, wobei die Zusammensetzung außerdem einen oder mehrere zusätzliche Bestandteile umfasst, die aus nichtionischen, anionischen, kationischen und amphoteren Tensiden, Vitaminen und Provitaminen, darunter Panthenol, Lichtschutzmitteln, Füllstoffen, Farbmitteln, Perlglanzmitteln, Trübungsmitteln, Sequestriermitteln, filmbildenden Polymeren, kationischen, anionischen oder neutralen Polymeren, die von dem bzw. den Polysacchariden a) verschieden sind, assoziativen Polymeren, Weichmachern, Verdickern, Ölen, Antioxidantien, Antischaummitteln, Feuchtigkeitsspendern, Emollientien, Penetriermitteln, Parfümen und Konservierungsmitteln ausgewählt sind; vorzugsweise die Zusammensetzung außerdem ein oder mehrere nichtionische, anionische, kationische oder amphotere Tenside, kationische, anionische oder neutrale Polymere, die von dem bzw. den Polysacchariden a) verschieden sind, oder assoziative Polymere umfasst.

9. Verfahren nach einem der vorhergehenden Ansprüche, das einen zusätzlichen Schritt des Erhitzens umfasst, der bei einer Temperatur im Bereich von 100 °C bis 250 °C, vorzugsweise im Bereich von 150 bis 220 °C, bevorzugt im Bereich von 160 °C bis 220 °C, weiter bevorzugt im Bereich von 160 °C bis 200 °C, durchgeführt wird, wobei dieser Schritt vorzugsweise nach dem Aufbringen des bzw. der aminierten und oxidierten Polysaccharide mit mindestens einer Carboxy(lat)gruppe a) erfolgt; insbesondere der Erhitzungsschritt mit einem Glätteisen durchgeführt wird.

10. Wässrige Zusammensetzung, die bei einem pH-Wert zwischen 1 und 9,5 einschließlich vorliegt; insbesondere die Zusammensetzung bei saurem pH-Wert, spezieller zwischen 2 und 6 einschließlich, noch spezieller zwischen 2,5 und 5, vorzugsweise zwischen 3 und 4, vorliegt; umfassend ein oder mehrere aminierte und oxidierte Polysaccharide mit mindestens einer Carboxygruppe -C(O)-OH oder Carboxylatgruppe -C(O)O-, M⁺, wobei M⁺ für ein kationisches Gegenion steht, a) gemäß einem der Ansprüche 1 bis 6 und 8.

11. Verwendung eines oder mehrerer aminierter und oxidierter Polysaccharide mit mindestens einer Carboxygruppe -C(O)-OH oder Carboxylatgruppe -C(O)O-, M⁺, wobei M⁺ für ein kationisches Gegenion steht, a) gemäß einem der Ansprüche 1 bis 5 zur Verbesserung der Konditionierung von Keratinfasern und/oder zum Bändigen oder Reparieren von beschädigten Keratinfasern und/oder zur Verhinderung des Brechens von Keratinfasern.

12. Verwendung einer Zusammensetzung gemäß Anspruch 10 zur Verbesserung der Konditionierung von Keratinfasern und/oder zum Bändigen oder Reparieren von beschädigten Keratinfasern und/oder zur Verhinderung des Brechens von Keratinfasern.

13. Kit oder Vorrichtung mit mehreren Kompartimenten, umfassend:
• eine erste Zusammensetzung gemäß Anspruch 10 und
• gegebenenfalls eine Vorrichtung zum Erhitzen der Keratinfasern auf eine Temperatur von mindestens 100 °C, vorzugsweise im Bereich von 100 bis 250 °C.

## Claims

1. Process for treating keratin materials, in particular keratin fibres, especially human keratin fibres, preferably the hair, comprising at least one step consisting in applying, to said fibres, a) one or more oxidized amino polysaccharide(s) comprising at least one carboxy group -C(O)-OH or carboxylate group -C(O)O⁻, M⁺ with M⁺ representing a cationic counterion, in which said oxidized polysaccharide(s) a) are represented by formula **(I)** below:
**(R₁R₂N)ₚ-P-(CHO)ₘ(COOX)ₙ** **(I)**
in which formula **(I):**
• **P** represents a polysaccharide chain constituted of monosaccharides comprising 5 carbon atoms or more than 5 carbon atoms, preferably 6 or more than 6 carbon atoms and more particularly 6 carbon atoms;
• **R₁** and **R₂** represent a hydrogen atom;
• **X** represents a hydrogen atom, a cationic counterion, in particular chosen from i) alkali metal, ii) alkaline earth metal, such as sodium or potassium, iii) ammonium R,R',R"N⁺- with R, R' and R", which may be identical or different, representing a hydrogen atom, a (C₁-C₆)alkyl group optionally substituted in particular with a hydroxyl group, such as hydroxyethyl; iv) and a cationic counterion derived from aqueous ammonia, monoethanolamine, diethanolamine, triethanolamine and 3-amino-1,2-propanediol, and basic amino acids such as lysine, arginine, sarcosine, ornithine and citrulline;
• **m + n** is greater than or equal to 1;
• **m** is such that the degree of substitution of the polysaccharide with one or more aldehyde groups (DS(CHO)), which is greater than or equal to 0, in particular m is within the range of from 0 to 2, more particularly ranging from 0.001 to 1.8, preferably ranging from 0.005 to 1.5;
• **n** is such that the degree of substitution of the polysaccharide with one or more carboxylic groups (DS(COOX)) is greater than 0, in particular less than or equal to 2, preferably between 0.001 and 1.5, inclusive;
• **p** is such that the degree of substitution of the polysaccharide with one or more amine groups (DS(R₁R₂N)) is greater than 0, in particular less than or equal to 2, preferably between 0.001 and 1.5, inclusive.

2. Process according to the preceding claim, in which the polysaccharide(s) a) are non-ionic or anionic.

3. Process according to either one of the preceding claims, in which m is within a range of from 0 to 1, n and **p,** which may be identical or different, are within a range of from 0.01 to 2.

4. Process according to Claim 3, **characterized in that** the polysaccharide chain is chosen from celluloses, starches, guar gums, inulins, xanthan gums, pullulan gums, agar-agar gums, carrageenan gums, gellan gums, gums arabic, tragacanth gums, xylans and derivatives thereof, cellobiose, maltodextrin, scleroglucan, chitosan, ulvan, fucoidan, alginate, pectin, heparin, hyaluronic acid, and cyclodextrin and derivatives thereof, and mixtures thereof.

5. Process according to Claim 3, **characterized in that** the polysaccharide chain is chosen from celluloses, (C₁-C₆) alkylcelluloses, hydroxy (C₁-C₆) alkylcelluloses such as hydroxyethylcelluloses and hydroxypropylcelluloses, carboxycelluloses and carboxy(C₁-C₆)alkylcelluloses such as carboxymethylcelluloses, starches, inulins, and preferably inulin.

6. Process according to any one of the preceding claims, in which the step of applying the oxidized amino polysaccharide(s) comprising at least one carboxy group -C(O)-OH or carboxylate group -C(O)O⁻, M⁺ with M⁺ representing a cationic counterion employs a composition comprising at least one oxidized amino polysaccharide comprising at least one carboxylate group as defined in any one of the preceding claims, in a content ranging from 0.05% to 15% by weight, relative to the total weight of the composition, in particular ranging from 0.1% to 10% by weight, more preferentially ranging from 0.2% to 6% by weight.

7. Process according to any one of the preceding claims, which employs a composition comprising at least one oxidized amino polysaccharide comprising at least one carboxylate group as defined in any one of the preceding claims, said composition being aqueous and at a pH of between 1 and 9.5, inclusive; in particular, said composition is at acidic pH, in addition particularly between 2 and 6, inclusive, even more particularly between 2.5 and 5, preferably between 3 and 4.

8. Process according to any one of the preceding claims, which employs a composition comprising at least one oxidized amino polysaccharide comprising at least one carboxylate group as defined in any one of Claims 1 to 6, said composition also comprising one or more additional ingredients chosen from non-ionic, anionic, cationic and amphoteric surfactants, vitamins and provitamins, including panthenol, sunscreens, fillers, colorants, nacreous agents, opacifiers, sequestrants, film-forming polymers, cationic, anionic or neutral polymers other than the polysaccharide(s) a), associated polymers, plasticizers, thickeners, oils, antioxidants, antifoams, moisturizers, emollients, penetrants, fragrances and preservatives; preferably, the composition also comprises one or more non-ionic, anionic, cationic and amphoteric surfactants, cationic, anionic or neutral polymers other than the polysaccharides a), or associative polymers.

9. Process according to any one of the preceding claims, which comprises an additional heating step which is carried out at a temperature ranging from 100 to 250°C, preferably ranging from 150 to 220°C, preferentially ranging from 160°C to 220°C, more preferentially ranging from 160°C to 200°C; preferably, this step takes place after the application of the oxidized amino polysaccharide(s) comprising at least one carboxy(late) group a); particularly, the heating step is carried out with a straightening iron.

10. Aqueous composition at a pH of between 1 and 9.5, inclusive, said composition being in particular at acidic pH, in addition particularly between 2 and 6, inclusive, even more particularly between 2.5 and 5, preferably between 3 and 4, comprising one or more oxidized amino polysaccharide(s) comprising at least one carboxy group -C(O)-OH or carboxylate group -C(O)O⁻, M⁺ with M⁺ representing a cationic counterion a) as defined in any one of Claims 1 to 6 and 8.

11. Use of one or more oxidized amino polysaccharide(s) comprising at least one carboxy group -C(O)-OH or carboxylate group -C(O)O⁻, M⁺ with M⁺ representing a cationic counterion a) as defined in any one of Claims 1 to 5, for improving the conditioning of keratin fibres and/or for making manageable and/or for repairing damaged keratin fibres, and/or for preventing the breaking of keratin fibres.

12. Use of a composition as defined in Claim 10, for improving the conditioning of keratin fibres and/or for making manageable and/or for repairing damaged keratin fibres, and/or for preventing the breaking of keratin fibres.

13. Multicompartment kit or device comprising:
• a first composition as defined in Claim 10; and
• optionally, a device for heating the keratin fibres to a temperature of at least 100°C, preferably ranging from 100 to 250°C.
